(19) Europäisches Patentamt
European Patent Office
Office européen des brevets

(11) Numéro de publication: **0 271 404 B1**

(12) # FASCICULE DE BREVET EUROPEEN

(45) Date de publication de fascicule du brevet: **14.08.91**

(51) Int. Cl.[5]: **C07D 401/04**, C07D 401/14, C07D 487/04, A61K 31/47, C07D 471/04

(21) Numéro de dépôt: **87402708.9**

(22) Date de dépôt: **01.12.87**

Le dossier contient des informations techniques présentées postérieurement au dépôt de la demande et ne figurant pas dans le présent fascicule.

(54) **Nouveaux dérivés du pyrrole, leur préparation et les compositions pharmaceutiques qui les contiennent.**

(30) Priorité: **02.12.86 FR 8616796**

(43) Date de publication de la demande: **15.06.88 Bulletin 88/24**

(45) Mention de la délivrance du brevet: **14.08.91 Bulletin 91/33**

(84) Etats contractants désignés: **AT BE CH DE ES FR GB GR IT LI LU NL SE**

(56) Documents cités:
**EP-A- 91 241**
**EP-A- 274 929**
**EP-A- 274 930**
**EP-A- 0 174 858**
**FR-A- 2 101 081**
**FR-A- 2 115 045**
**GB-A- 1 358 680**
**GB-A- 1 397 060**
**GB-A- 1 466 829**
**GB-A- 1 468 497**

**LIEBIGS ANNALEN DER CHEMIE, 1985, pages 1679-1691, VCH Verlags Gmbh,Weinheim,DE L.SOMOGYI:"Isoindoline derivatives of opianic acid"**

(73) Titulaire: **RHONE-POULENC SANTE**
**20, avenue Raymond Aron**
**F-92160 Antony(FR)**

(72) Inventeur: **Bourzat, Jean-Dominique**
**35, rue Santos-Dumont**
**F-75015 Paris(FR)**
Inventeur: **Capet, Marc**
**10, rue de la Galaise**
**F-94320 Thiais(FR)**
Inventeur: **Cotrel, Claude**
**17 A avenue du Dr. Arnold Netter**
**F-75012 Paris(FR)**
Inventeur: **Labaudinière, Richard**
**11, rue du Château**
**F-94400 Vitry sur Seine(FR)**
Inventeur: **Pitchen, Philippe**
**31, avenue de la Belle Image**
**F-94440 Marolles-en-Brie(FR)**
Inventeur: **Roussel, Gérard**
**20 ter, rue des Carrières**
**F-91450 Soisy sur Seine(FR)**

**EP 0 271 404 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

(74) Mandataire: **Pilard, Jacques et al**
**RHONE-POULENC SANTE, Service Brevets,**
**20 Avenue Raymond Aron**
**F-92165 Antony Cédex(FR)**

## Description

La présente invention concerne de nouveaux dérivés du pyrrole de formule générale :

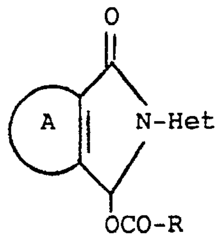

(I)

dans laquelle A forme avec le cycle pyrrole un noyau isoindoline, et Het représente un radical naphtyridinyle substitué par un atome d'halogène ou un radical alcoyle (1 à 4 C), alcoyloxy (1 à 4 C) et R représente un radical alcoyle non substitué ou substitué par un radical alcoyloxy, dialcoylamino, alcoylcarbonylamino (dont la partie amino peut être éventuellement substituée par un radical alcoyle), pipéridyle ou pipéridino, ou bien R représente un radical pipéridyle-4, étant entendu que les radicaux alcoyle sont en chaîne droite ou ramifiée et contiennent, sauf mention spéciale, 1 à 10 atomes de carbone et que les radicaux pipéridino, pipéridyle peuvent être non substitués ou substitués en position quelconque par un radical alcoyle, ainsi que, lorsqu' ils existent, leurs sels pharmaceutiquement acceptables et les isomères optiques des produits de formule (I).

Comme produits possédant des propriétés tranquillisantes et anticonvulsivantes sont connus des dérivés de l'isoindoline, de la pyrrolo[3,4-b] pyrazine, du dithiino[2,3-c] pyrrole ou de l'oxathiino[2,3-c] pyrrole qui sont décrits dans les brevets français FR 2 101 081 et 2 115 045 ou anglais GB 1 468 497. Dans la demande de brevet européen EP 91241 sont décrits des produits de structure analogue présentant des propriétés anxiolytiques.

Dans les demandes de brevets européens EP-A-274 929 et EP-A-274 930 sont décrits des composés structurellement très proches des produits qui font l'objet de la présente invention et qui présentent le même type d'activité.

Selon l'invention, les produits de formule générale (I) dans laquelle Het est défini comme précédemment à l'exception de représenter un radical naphtyridine-1,8 yle-2 substitué en position 7 par un radical alcoyloxy et les autres symboles sont définis comme précédemment, peuvent être préparés par action d'un acide de formule générale :

R-COOH (II)

ou un sel alcalin de cet acide, dans laquelle R est défini comme précédemment sur un produit de formule générale :

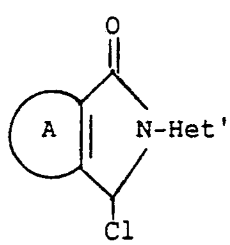

(III)

dans laquelle Het' a les significations données précédemment pour Het à l'exception de représenter un radical naphtyridine-1,8 yle-2 substitué en position 7 par un radical alcoyloxy et A est défini comme précédemment.

On opère généralement en présence d'un agent de condensation tel que le diaza-1,8 bicyclo [5.4.0] undécène-7 ou le diaza-1,5 bicyclo [5.3.0] nonène-5 ou un hydroxyde d'ammonium quaternaire tel que l'hydroxyde de triéthylbenzylammonium, dans un solvant organique tel que le diméthylformamide à une température comprise entre 20 et 100°C ou simplement, lorsqu'on emploie le sel alcalin de l'acide, dans le

diméthylformamide à une température de 20° C.

Les produits de formule générale (III) peuvent être préparés par chloruration d'un produit de formule générale :

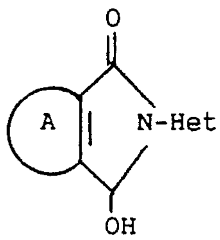

(IV)

dans laquelle A et Het sont définis comme précédemment.

On opère généralement en présence d'un agent de chloruration tel que le chlorure de sulfinyle ou l'oxychlorure de phosphore en présence de quantités catalytiques de diméthylformamide à une température comprise entre 20° C et la température de reflux du mélange réactionnel ou de tout autre agent connu de l'homme du métier permettant de transformer un radical hydroxy en radical chloro sans toucher au reste de la molécule.

Les produits de formule générale (IV) peuvent être préparés par application ou adaptation des méthodes décrites dans les brevets belges 815 019 ou 835 325.

Selon l'invention, les produits de formule générale (I) peuvent également être préparés par action d'un dérivé de formule générale :

RCO-X (V)

dans laquelle R est défini comme précédemment et X représente un atome d'halogène tel que le chlore, ou bien représente un reste activé tel qu'un radical imidazolyle-1 ou un radical R"CO-O- dans lequel R" représente un radical alcoyle, sur un dérivé de formule générale (IV) défini comme précédemment.

On opère généralement dans un solvant organique tel que le chloroforme ou le chlorure de méthylène ou un éther comme le tétrahydrofuranne ou le dioxanne ou bien dans le diméthylformamide, à une température comprise entre 0° C et la température de reflux du mélange réactionnel en présence d'une base telle que l'hydrure de sodium ou un accepteur d'acide tel que la triéthylamine ou la pyridine.

Selon l'invention, les produits de formule générale (I) peuvent être également préparés par action d'un sel alcalin d'un acide de formule générale (II) sur un produit de formule générale :

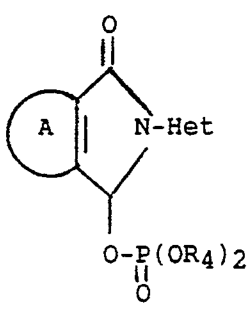

(VI)

dans laquelle $R_4$ représente un radical alcoyle contenant 1 à 4 atomes de carbone en chaîne droite ou ramifiée ou un radical phényle et A et Het sont définis comme précédemment.

On opère généralement dans un solvant organique tel que le diméthylformamide à une température comprise entre 0 et 25° C.

Les produits de formule générale (VI) peuvent être préparés par action d'un produit de formule générale :

$$Cl-\underset{\underset{O}{\parallel}}{P}(OR_4)_2 \qquad (VII)$$

dans laquelle $R_4$ représente un radical alcoyle contenant 1 à 4 atomes de carbone en chaîne droite ou ramifiée ou un radical phényle, sur un produit de formule générale (IV) dans laquelle Het et A sont définis comme précédemment.

On opère généralement dans un solvant organique tel que le diméthylformamide en présence d'une base telle qu'un hydrure de métal alcalin comme l'hydrure de sodium à une température comprise entre -5 et + 25 °C.

Il n'est pas nécessaire d'isoler le produit de formule générale (VI) pour mettre en oeuvre le procédé selon l'invention. Il suffit d'effectuer la condensation des produits de formule générale (VII) et (IV) comme il vient d'être dit, puis d'ajouter le sel alcalin de l'acide de formule générale (II) dans le mélange réactionnel.

Comme l'homme du métier pourra s'en rendre compte, certains radicaux entrant dans la définition du symbole R sont incompatibles avec des réactifs mis en oeuvre au cours des réactions et doivent être protégés préalablement à la mise en oeuvre des procédés ou de certaines phases des procédés exposés précédemment. C'est notamment le cas lorsque le radical R contient des fonctions amines primaires ou secondaires ou des fonctions hydroxylées susceptibles de donner naissance à des réactions secondaires. Dans ce cas les-dites fonctions devront être protégées par toute méthode connue de l'homme du métier puis être débloquées après réaction.

Les nouveaux produits de formule générale (I) peuvent être purifiés par les méthodes connues habituelles, par exemple par cristallisation, chromatographie ou extractions successives en milieu acide et basique.

Les nouveaux produits de formule générale (I) peuvent être transformés en sel d'addition avec les acides par action d'un acide dans un solvant organique tel qu'un alcool, une cétone, un éther ou un solvant chloré. Le sel formé précipite, éventuellement après concentration de sa solution ; il est séparé par filtration ou décantation.

Les produits de formule générale (I) présentent des propriétés pharmacologiques particulièrement intéressantes révélatrices d'une activité anxiolytique, hypnotique, anticonvulsivante, antiépileptique et myorelaxante. C'est ainsi qu'ils présentent une bonne affinité in vitro pour les sites récepteurs à benzodiazépine à des concentrations dont les valeurs sont comprises entre 0,4 et 200 nM selon la technique décrite par J.C. BLANCHARD et L. JULOU, J. of Neurochemistry, 40, 601 (1983) inspirée des travaux de SQUIRES et BRAESTRUP, Nature, 266, 732 (1977).

Chez l'animal (souris) ils se sont montrés actifs à des doses généralement comprises entre 0,3 et 200 mg/kg par voie orale vis-à-vis des convulsions induites par le pentétrazol selon une technique voisine de celle de EVERETT et RICHARDS, J. Pharmacol., 81, 402 (1944).

Les nouveaux produits de formule générale (I) et leurs sels présentent en outre une toxicité faible. Leur $DL_{50}$ est généralement comprise entre 300 et 900 mg/kg, par voie orale chez la souris.

Pour l'emploi médicinal, il peut être fait usage des nouveaux produits de formule générale (I) tels quels ou à l'état de sels pharmaceutiquement acceptables, c'est-à-dire non toxiques aux doses d'utilisation.

Comme exemples de sels pharmaceutiquement acceptables on peut citer les sels d'addition avec les acides minéraux tels que chlorhydrates, sulfates, nitrates, phosphates ou organiques tels que acétates, propionates, succinates, benzoates, fumarates, maléates, méthanesulfonates, iséthionates, théophyllineacétates, salicylates, phénolphtalinates, méthylène-bis-$\beta$-oxynaphtoates ou des dérivés de substitution de ces composés.

Sont particulièrement intéressants les produits de formule générale (I) dans laquelle A forme avec le cycle pyrrole un noyau isoindoline et Het représente un radical naphtyridine-1,8 yl-2 substitué par un atome d'halogène ou un radical alcoyloxy (1 à 4 C) et R représente un radical alcoyle contenant 1 à 6 atomes de carbone en chaîne droite ou ramifiée non substitué ou substitué par un radical alcoyloxy, dialcoylamino, alcoylcarbonylamino, pipéridino ou bien R représente un radical pipéridyle-3, étant entendu que les radicaux alcoyle sont en chaîne droite ou ramifiée et contiennent, sauf mention spéciale, 1 à 10 atomes de carbone et que les radicaux pipéridino, pipéridyle peuvent être non substitués ou substitués en position quelconque par un ou plusieurs radicaux alcoyle.

Sont d'un intérêt tout particulier, les produits suivants :

- Acétamido-4 butyrate de (chloro-7 naphtyridine-1,8 yl-2)-2 oxo-3 isoindolinyle-1-(RS)
- (Propionyl-1 pipéridyl-4) carboxylate de (chloro-7 naphtyridine-1,8 yl-2)-2 oxo-3 isoindolinyle-1-(RS)
- Méthyl-5 hexanoate de (méthoxy-7 naphtyridine-1,8 yl-2)-2 oxo-3 isoindolinyle-1-(RS).

- Diisopropylamino-3 propionate de (chloro-7 naphtyridine-1,8 yl-2)-2 oxo-3 isoindolinyle-1-(RS)

Les exemples suivants montrent comment l'invention peut être mise en pratique.

EXEMPLE 1

A une solution de 16,5 g de chloro-3 (chloro-7 naphtyridine-1,8 yl-2)-2 isoindolinone-1 dans 100 cm3 de diméthylformamide anhydre, maintenue sous atmosphère d'argon, on ajoute, à une température voisine de 20° C, 9,2 g de chlorhydrate de l'acide diméthylamino-3 propionique et 16,7 g de diaza-1,8 bicyclo [5.4.0] undécène-7 et agite la suspension obtenue pendant 24 heures à une température voisine de 20° C. On ajoute alors 200 cm3 d'eau distillée et 200 cm3 de dichlorométhane. La phase aqueuse est séparée par décantation puis réextraite par 3 fois 50 cm3 de dichlorométhane. Les phases organiques sont réunies, lavées par 2 fois 50 cm3 d'eau distillée, séchées sur du sulfate de magnésium, filtrées puis concentrées à sec sous pression réduite (2,7 kPa) à 80° C. Le résidu obtenu est dissous dans 100 cm3 de dichlorométhane et la solution est extraite par 2 fois 100 cm3 d'une solution aqueuse d'acide chlorhydrique 1N. Les phases aqueuses sont réunies, lavées par 50 cm3 de dichlorométhane, alcalinisées par une solution de soude 10N jusqu'à un pH voisin de 11 et extraites par 2 fois 150 cm3 de dichlorométhane. Les phases organiques sont réunies, lavées par 2 fois 30 cm3 d'eau distillée, séchées sur du sulfate de magnésium, filtrées puis concentrées à sec sous pression réduite (2,7 kPa) à 60° C. Après deux recristallisations successives dans l'éthanol du produit ainsi obtenu, on obtient 2,9 g de diméthylamino-3 propionate de (chloro-7 naphtyridine-1,8 yl-2)-2 oxo-3 isoindolinyle-1 fondant à 150° C.

Le chlorhydrate de l'acide diméthylamino-3 propionique peut être obtenu par la méthode décrite par CLARKE H.T., GILLESPIE H.B., WEISSHAUS S.Z., J. Am. Chem. Soc., 55, 4571 (1933).

La chloro-3 (chloro-7 naphtyridine-1,8 yl-2)-2 isoindolinone-1 peut être préparée de la manière suivante : A 15,5 g d'hydroxy-3 (méthoxy-7 naphtyridine-1,8 yl-2)-2 isoindolinone-1, on ajoute goutte à goutte sous agitation 200 cm3 de chlorure de sulfinyle. Le mélange réactionnel est chauffé à reflux sous agitation, pendant 1 heure puis est additionné de 10,5 cm3 de diméthylformamide et chauffé à nouveau à reflux pendant 3 heures, puis refroidi à une température voisine de 60° C et concentré à sec sous pression réduite (2,7 kPa) à 60° C. Au résidu obtenu, on ajoute 100 cm3 de dichlorométhane et concentre le mélange à sec sous pression réduite (2,7 kPa) à 60° C. Au solide résiduel obtenu, on ajoute 100 cm3 de dichlorométhane et agite pendant 10 minutes. Le produit insoluble est séparé par filtration et lavé par 15 cm3 de dichlorométhane puis par 2 fois 25 cm3 d'oxyde de diisopropyle et séché à l'air. On obtient ainsi 12,4 g de chloro-3 (chloro-7 naphtyridine-1,8 yl-2)-2 isoindolinone-1, infusible à 300° C.

L'hydroxy-3 (méthoxy-7 naphtyridine-1,8 yl-2)-2 isoindolinone-1 peut être préparée par la méthode décrite dans le brevet belge 815 019.

EXEMPLE 2

En opérant d'une manière analogue à celle décrite à l'exemple 1, mais à partir de 8,25 g de chloro-3 (chloro-7 naphtyridine-1,8 yl-2)-2 isoindolinone-1, de 4,25 g de chlorhydrate de l'acide diméthylamino-4 butanoïque et de 7,6 g de diaza-1,8 bicyclo [5.4.0] undécène-7, on obtient 4,1 g de diméthylamino-4 butanoate de (chloro-7 naphtyridine-1,8 yl-2)-2 oxo-3 isoindolinyle-1, fondant à 148° C.

L'acide diméthylamino-4 butanoïque peut être préparé par la méthode décrite par C. HARRIES, F. DUVEL, Liebigs Ann. Chem., (1915) 410, 54.

EXEMPLE 3

A une solution de 6,6 g de chloro-3 (chloro-7 naphtyridine-1,8 yl-2)-2 isoindolinone dans 60 cm3 de diméthylformamide anhydre maintenue sous atmosphère d'argon, on ajoute à une température voisine de 20° C, 2,4 g d'acide méthyl-4 pentanoïque et 3,05 g de diaza-1,8 bicyclo [5.4.0] undécène-7 et on agite la suspension obtenue pendant 24 heures à une température voisine de 20° C. On ajoute alors 500 cm3 d'eau distillée et 150 cm3 de dichlorométhane. La phase aqueuse est séparée par décantation puis réextraite par 2 fois 150 cm3 de dichlorométhane. Les phases organiques sont réunies, lavées par 3 fois 50 cm3 d'eau distillée, séchées sur du sulfate de magnésium, filtrées puis concentrées à sec sous pression réduite (2,7 kPa) à 60° C. Le résidu huileux est purifié par chromatographie sur 150 g de gel de silice contenus dans une colonne de 3,5 cm de diamètre [éluant : dichlorométhane-méthanol (98-2 en volumes)]. On élue d'abord avec 200 cm3 de solvant : l'éluat correspondant est éliminé ; on élue ensuite avec 900 cm3 de solvant : l'éluat correspondant est concentré à sec sous pression réduite (2,7 kPa) à 40° C. Après recristallisation dans l'éthanol, on obtient 4 g de méthyl-4 pentanoate de (chloro-7 naphtyridine-1,8 yl-2)-2

oxo-3 isoindolinyle-1, fondant à 147° C.

EXEMPLE 4

En opérant comme à l'exemple 1 mais à partir de 9,9 g de chloro-3 (chloro-7 naphtyridine-1,8 yl-2)-2 isoindolinone-1 dans 100 cm3 de diméthylformamide anhydre, de 5,4 g de chlorhydrate de l'acide méthyl-1 pipéridine-3 carboxylique et de 10,7 g de diaza-1,8 bicyclo [5.4.0] undécène-7, on obtient, après précipitation dans 1000 cm3 d'eau, filtration et séchage à l'air, 11,3 g d'un produit fondant vers 70° C. Le solide obtenu est dissous dans 40 cm3 d'éthanol. A la solution chaude obtenue, on ajoute une solution de 3 g d'acide fumarique dans 30 cm3 d'éthanol. Le produit cristallisé obtenu est séparé par filtration, lavé par 15 cm3 d'éthanol et séché sous pression réduite (0,07 kPa) à 45° C. On obtient ainsi 9,8 g de fumarate acide de méthyl pipéridine-3 carboxylate de (chloro-7 naphtyridine-1,8 yl-2)-2 oxo-3 isoindolinyle-1 fondant à 211° C.

Le chlorhydrate de l'acide (méthyl-1 pipéridine-3) carboxylique peut être préparé de la façon suivante : 17,1g de (méthyl-1 pipéridine-3) carboxylate d'éthyle sont dissous dans 67 cm3 d'une solution aqueuse d'acide chlorhydrique 6N. Après 6 heures à reflux, la solution est concentrée à sec et le résidu recristallisé dans l'acétone. On obtient ainsi 15,7 g de chlorhydrate de l'acide (méthyl-1 pipéridine-3) carboxylique fondant à 186° C.

EXEMPLE 5

En opérant comme à l'exemple 1 mais à partir de 9,9g de chloro-3 (chloro-7 naphtyridine-1,8 yl-2)-2 isoindolinone-1 dans 100 cm3 de diméthylformamide anhydre, de 5,4 g de chlorhydrate de l'acide méthyl-1 pipéridine-4 carboxylique et de 10,7 g de diaza-1,8 bicyclo [5.4.0] undécène-7, et après deux recristallisations successives du résidu obtenu dans l'éthanol puis dans l'acétonitrile, on obtient 5,6 g de méthyl-1 pipéridinecarboxylate-4 de (chloro-7 naphtyridine-1,8 yl-2)-2 oxo-3 isoindolinyle-1 fondant à 136° C puis à 157° C.

Le chlorohydrate de l'acide méthyl-1 pipéridine-4 carboxylique peut être préparé selon la méthode décrite à l'exemple 4 pour la préparation du chlorhydrate de l'acide méthyl-1 pipéridine-3 carboxylique mais à partir de 8,6 g de méthyl-1 pipéridine-4 carboxylate d'éthyle et 33 cm3 d'une solution aqueuse d'acide chlorhydrique 6N. Après recristallisation dans l'acétone, on obtient 6,5 g de chlorhydrate de l'acide méthyl pipéridine-4 carboxylique fondant à 231 ° C.

Le méthyl-1 pipéridine-4 carboxylate d'éthyle peut être préparé de la façon suivante : A une solution de 15,7 g de pipéridine-4 carboxylate d'éthyle dans 8 cm3 d'eau, maintenue à une température voisine de 5° C, on ajoute en 15 minutes à la même température 20,3 cm3 d'une solution à 37 % (poids/volume) de formaldéhyde puis à nouveau en 15 minutes 11,5 g d'acide formique. On chauffe pendant 4 heures à reflux puis on refroidit et amène le mélange jusqu'à pH voisin de 10 à l'aide d'une solution aqueuse de soude 10N. Après extraction par 3 fois 150 cm3 de chlorure de méthylène, lavage à l'eau des extraits organiques, séchage et concentration à sec sous préssion réduite (2,7 kPa) à 70° C, on obtient 13,5 g de méthyl-1 pipéridine-4 carboxylate d'éthyle à l'état d'huile employée brute dans les synthèses ultérieures.

EXEMPLE 6

En opérant comme à l'exemple 1 mais à partir de 6,9 g de chloro-3 (chloro-7 naphtyridine-1,8 yl-2)-2 isoindolinone-1 dans 70 cm3 de diméthylformamide anhydre, de 4,4 g de chlorhydrate de l'acide diisopropylamino-3 propionique et de 7,45 g de diaza-1,8 bicyclo[5.4.0] undécène-7 et après recristallisations successives du résidu obtenu d'abord dans l'acétonitrile puis dans l'éthanol, on obtient 2,7 g de diisopropylamino-3 propionate de (chloro-7 naphtyridine-1,8 yl-2)-2 oxo-3 isoindolinyle-1 fondant à 135° C.

Le chlorhydrate de l'acide diisopropylamino-3 propionique peut être obtenu en opérant selon la méthode décrite à l'exemple 4 pour la préparation de l'acide méthyl-1 pipéridine-3 carboxylique mais à partir de 5 g de diisopropylamino-3 propionate d'éthyle et de 35 cm3 d'une solution aqueuse d'acide chlorhydrique 6N. Après recristallisation du produit obtenu dans l'acétone, on obtient 2,3 g de chlorhydrate de l'acide diisopropylamino-3 propionique fondant à 170° C.

Le diisopropylamino-3 propionate d'éthyle peut être obtenu de la façon suivante : On introduit, goutte à goutte, en 30 minutes, 18,1 g de bromo-3 propionate d'éthyle dans une solution de 28,5 cm3 de diisopropylamine et de 40 cm3 d'éthanol maintenue à une température de 25° C. La suspension obtenue est chauffée à reflux pendant 4 heures. Après refroidissement, le mélange réactionnel est repris par 100 cm3 d'eau et 70 cm3 de solution aqueuse d'acide chlorhydrique 4N. Après lavage par 100 cm3 d'éther

éthylique, on alcalinise à un pH voisin de 9 par une solution aqueuse de soude 4N. L'huile formée est extraite par 3 fois 150 cm3 de chlorure de méthylène. Après lavage par 2 fois 100 cm3 d'eau et séchage, la solution chlorométhylénique obtenue est concentrée à sec sous pression réduite (2,7 kPa) à 40° C. On obtient ainsi 11,6 g de diisopropylamino-3 propionate d'éthyle à l'état d'huile employée brute dans les synthèses ultérieures.

EXEMPLE 7

A une solution de 12,3 g de (méthoxy-7 naphtyridine-1,8 yl-2)-2 hydroxy-3 isoindolinone-1 dans 200 cm3 de chlorure de méthylène maintenue à une température voisine de 20° C, on ajoute 27 cm3 de triéthylamine. On introduit ensuite, goutte à goutte, en 20 minutes, 10,8 g de chlorure de méthyl-4 pentanoyle et 50 mg de diméthylamino-4 pyridine puis on chauffe le mélange réactionnel pendant 19 heures à reflux. La suspension obtenue est versée dans 800 cm3 d'eau et le solide obtenu est séparé par filtration et éliminé. La phase organique est décantée, lavée à l'eau, séchée et concentrée à sec sous pression réduite (2,7 kPa). Le résidu huileux obtenu est purifié par chromatographie sur 150 g de gel de silice (0,063-0,2 mm) contenus dans une colonne de 2,7 cm de diamètre (éluant : chlorure de méthylène) en éluant des fractions de 50 cm3. Les fractions 6 à 18 sont réunies et concentrées à sec sous pression réduite (2,7 kPa) à 40° C. Après recristallisation dans l'acétonitrile du solide obtenu, on obtient 4,9 g de méthyl-4 pentanoate de (méthoxy-7 naphtyridine-1,8 yl-2)-2 oxo-3 isoindolinyle-1 fondant à 133° C.

Le chlorure de méthyl-4 pentanoyle peut être préparé selon F. KOGL et C.A. SALEMINK, Rec. Trav. Chim, 71, 779-97 (1952).

EXEMPLE 8

En opérant comme à l'exemple 1 mais à partir de 9,9 g de chloro-3 (chloro-7 naphtyridine-1,8 yl-2)-2 isoindolinone-1 dans 100 cm3 de diméthylformamide anhydre, de 3,9 g d'acide méthyl-5 hexanoïque et de 4,6 g de diaza-1,8 bicyclo [5.4.0] undécène-7 et après recristallisation dans l'éthanol, on obtient 8 g de méthyl-5 hexanoate de (chloro-7 naphtyridine-1,8 yl-2)-2 oxo-3 isoindolinyle-1 fondant à 132° C.

EXEMPLE 9

On opère comme à l'exemple 7 mais à partir de 6,8 g de (méthoxy-7 naphtyridine-1,8 yl-2)-2 hydroxy-3 isoindolinone-1 dans 100 cm3 de chlorure de méthylène, de 10,1 g de triéthylamine, de 6,4 g de chlorure de méthyl-5 hexanoyle et de 50 mg de diméthylamino-4 pyridine. Le résidu obtenu après traitement, est purifié par chromatographie sur 100 g de gel de silice (0,063-0,2 mm) contenus dans une colonne de 2,8 cm de diamètre (éluant : chlorure de méthylène) en recueillant des fractions de 30 cm3. Les fractions 19 à 94 sont réunies et concentrées à sec sous pression réduite (2,7 kPa). Après recristallisation du résidu obtenu dans 75 cm3 d'heptane, on obtient 5,6 g de méthyl-5 hexanoate de (méthoxy-7 naphtyridine-1,8 yl-2)-2 oxo-3 isoindolinyle-1 fondant à 105° C.

EXEMPLE 10

En opérant comme à l'exemple 1 mais à partir de 29,7 g de chloro-3 (chloro-7 naphtyridine-1,8 yl-2)-2 isoindolinone-1 dans 300 cm3 de diméthylformamide anhydre, de 13,1 g d'acide acétamido-4 butyrique et de 13,7 g de diaza-1,8 bicyclo [5.4.0] undécène-7, on obtient, après recristallisation dans l'acétonitrile, 18 g d'acétamido-4 butyrate de (chloro-7 naphtyridine-1,8 yl-2)- 2 oxo-3 isoindolinyle-1 fondant à 186° C.

EXEMPLE 11

On opère comme à l'exemple 1 mais à partir de 4,95 g de chloro-3 (chloro-7 naphtyridine-1,8 yl-2)-2 isoindolinone-1 dans 60 cm3 de diméthylformamide anhydre, de 2,05 g d'acide phénylacétique et de 2,25 g de diaza-1,8 bicyclo [5.4.0] undécène-7. Après recristallisation dans l'acétate d'éthyle, on obtient 4,4 g de phénylacétate de (chloro-7 naphtyridine-1,8 yl-2)-2 oxo-3 isoindolinyle-1 fondant à 222-224° C.

EXEMPLE 12

En opérant comme à l'exemple 1 mais à partir de 9,9 g de chloro-3 (chloro-7 naphtyridine-1,8 yl-2)-2 isoindolinone-1 dans 100 cm3 de diméthylformamide anhydre, de 6,7 g de chlorhydrate de l'acide

(diméthyl-2,6 pipéridino)-3 propionique et de 10,7 g de diaza-1,8 bicyclo [5.4.0] undécène-7, on obtient, après recristallisation dans l'éthanol, 6 g de (diméthyl-2,6 pipéridino)-3 propionate de (chloro-7 naphtyridine-1,8 yl-2)-2 oxo-3 isoindolinyle-1 fondant à 159 °C.

Le chlorhydrate de l'acide (diméthyl-2,6 pipéridino)-3 propionique peut être obtenu en opérant selon la méthode décrite à l'exemple 4 pour la préparation du chlorhydrate de l'acide méthyl-1 pipéridine-3 carboxylique mais à partir de 12,5 g de chlorhydrate de (diméthyl-2,6 pipéridino)-3 propionate d'éthyle et de 35 cm3 d'une solution aqueuse d'acide chlorhydrique 6N. On obtient ainsi le chlorhydrate de l'acide (diméthyl-2,6 pipéridino)-3 propionique fondant à 215 °C.

Le chlorhydrate de (diméthyl-2,6 pipéridino)-3 propionate d'éthyle peut être obtenu en opérant selon la méthode décrite à l'exemple 6 pour la préparation du diisopropylamino-3 propionate d'éthyle mais à partir de 18,1 g de bromo-3 propionate d'éthyle, de 27 cm3 de diméthyl-2,6 pipéridine et de 30 cm3 d'éthanol. Le mélange réactionnel est concentré à sec sous pression réduite (2,7 kPa) et le résidu obtenu est repris par 50 cm3 d'eau et 30 cm3 d'une solution aqueuse d'acide chlorhydrique 4N. La phase aqueuse est lavée par 2 fois 80 cm3 d'éther éthylique, et neutralisée par 40 cm3 d'une solution aqueuse de soude 4N. L'huile insoluble est extraite par 3 fois 120 cm3 d'éther éthylique ; les extraits organiques sont ensuite lavés par 2 fois 80 cm3 d'eau et concentrés à sec sous pression réduite (2,7 kPa) à 40 °C. Le résidu est dissous dans 100 cm3 d'éther éthylique. A la solution obtenue, on ajoute 13,4 cm3 d'une solution 4,5N d'acide chlorhydrique gazeux dans l'éther éthylique. Un produit précipite. Il est séparé par filtration, lavé et séché à l'air. On obtient ainsi 14,4 g de chlorhydrate de (diméthyl-2,6 pipéridino)-3 propionate d'éthyle fondant à 146 °C.

EXEMPLE 13

En opérant comme à l'exemple 1 mais à partir de 9,9 g de chloro-3 (chloro-7 naphtyridine-1,8 yl-2)-2 isoindolinone-1 dans 100 cm3 de diméthylformamide anhydre, de 6,2 g de chlorhydrate de l'acide pipéridino-4 butyrique et de 10,7 g de diaza-1,8 bicyclo [5.4.0] undécène-7, on obtient, après recristallisation dans l'éthanol, 9,4 g de pipéridino-4 butyrate de (chloro-7 naphtyridine-1,8 yl-2)-2 oxo-3 isoindolinyle-1 fondant à 166 °C.

Le chlorhydrate de l'acide pipéridino-4 butyrique peut être obtenu en opérant selon la méthode décrite à l'exemple 4 pour la préparation du chlorhydrate de l'acide méthyl-1 pipéridine-3 carboxylique, mais à partir de 19,9 g de pipéridino-4 butyrate d'éthyle et de 66,5 cm3 d'une solution aqueuse d'acide chlorhydrique 6N et en chauffant 24 heures à reflux. On obtient ainsi 16,3 g de chlorhydrate de l'acide pipéridino-4 butyrique fondant à 190 °C.

Le pipéridino-4 butyrate d'éthyle peut être préparé en opérant selon la méthode décrite à l'exemple 6 pour la préparation du diisopropylamino-3 propionate d'éthyle, mais à partir de 48,8 g de bromo-4 butyrate d'éthyle, de 42,5 g de pipéridine et de 75 cm3 d'éthanol. Le mélange réactionnel est repris par 200 cm3 d'eau et 120 cm3 d'une solution aqueuse d'acide chlorhydrique 4N. Après lavage par 150 cm3 d'éther éthylique, la phase aqueuse est alcalinisée à un pH voisin de 9 par une solution aqueuse de soude 4N. L'huile formée est extraite par 3 fois 150 cm3 de chlorure de méthylène. Les extraits organiques sont réunis, lavés à l'eau, séchés et concentrés à sec sous pression réduite (2,7 kPa) à 70 °C. On obtient ainsi 47 g de pipéridino-4 butyrate d'éthyle à l'état d'huile employée brute dans les synthèses ultérieures.

EXEMPLE 14

En opérant comme à l'exemple 1 mais à partir de 9,9 g de chloro-3 (chloro-7 naphtyridine-1,8 yl-2)-2 isoindolinone-1 dans 100 cm3 de diméthylformamide anhydre, de 4,8 g d'acide propionamido-4 butanoïque et de 4,6 g de diaza-1,8 bicyclo [5.4.0] undécène-7, on obtient, après recristallisation dans l'acétonitrile, 6,6 g de propionamido-4 butyrate de (chloro-7 naphtyridine-1,8 yl-2)-2 oxo-3 isoindolinyle-1 fondant à 179 °C.

L'acide propionamido-4 butanoïque peut être préparé de la façon suivante : on ajoute, en 15 minutes, 10,3 g d'acide amino-4 butyrique dans 10 cm3 d'anhydride propionique à une température voisine de 20 °C, puis 5 gouttes d'acide sulfurique concentré (d = 1,83) et on chauffe à une température voisine de 100 °C pendant 2 heures. Après refroidissement, le solide cristallisé est séparé par filtration, lavé par 5 fois 100 cm3 d'éther éthylique et séché. On obtient ainsi 9,8 g d'acide propionamido-4 butanoïque fondant à 85-90 °C.

EXEMPLE 15

A une solution de 9,2 g de (méthoxy-7 naphtyridine-1,8 yl-2)-2 hydroxy-3 isoindolinone-1 et de 20 cm3

de triéthylamine dans 150 cm3 de dichloro-1,2 éthane maintenue à une température voisine de 20 ° C, on ajoute, en 20 minutes, une solution de 9 g de chlorure de méthyl-2 propoxyacétyle dans 20 cm3 de dichloro-1,2 éthane puis 50 mg de diméthylamino-4 pyridine et on chauffe à reflux pendant 16 heures. Le mélange réactionnel est versé dans 250 cm3 d'eau puis extrait par 100 cm 3 de chlorure de méthylène. Après lavage à l'eau, séchage et concentration à sec sous pression réduite (3 kPa), le résidu obtenu est purifié par deux recristallisations successives dans l'éthanol. On obtient ainsi 7,9 g de méthyl-2 propoxyacétate de (méthoxy-7 naphtyridine-1,8 yl-2)-2 oxo-3 isoindolinyle-1 fondant à 114 ° C.

Le chlorure de méthyl-2 propoxyacétyle peut être obtenu de la façon suivante : On ajoute, en 15 minutes, 5 cm3 de chlorure de thionyle à une solution de 8,3 g d'acide méthyl-2 propoxyacétique dans 50 cm3 de chloroforme. Le mélange est chauffé pendant 5 heures à reflux puis évaporé à sec sous pression réduite (2,7 kPa). On obtient ainsi 7,5 g de chlorure de méthyl-2 propoxyacétyle à l'état d'huile employée brute dans les synthèses ultérieures.

L'acide méthyl-2 propoxyacétique peut être obtenu de la façon suivante : A 200 cm3 d'alcool isobutylique maintenu à une température voisine de 100 ° C, on ajoute 12,7 g de sodium et chauffe jusqu'à disparition de celui-ci. On ajoute alors, en 1 heure, 23,6 g d'acide chloracétique et continue le chauffage pendant 2 heures. Après refroidissement, le mélange réactionnel est versé dans 250 cm3 d'eau. La phase aqueuse est lavée par 200 cm3 d'éther éthylique, concentrée à demi-volume sous pression réduite (3 kPa) puis acidifiée à un pH voisin de 1 avec une solution aqueuse d'acide chlorhydrique 1N. L'huile formée est extraite par 3 fois 150 cm3 d'éther éthylique. La phase organique est lavée à l'eau, séchée et concentrée à sec sous pression réduite (3 kPa). Après distillation sous pression réduite du résidu obtenu, on obtient 21,8 g d'acide méthyl-2 propoxyacétique bouillant à 92-96 ° C sous 0.93 kPa.

EXEMPLE 16

On opère comme à l'exemple 1 mais à partir de 6,6 g de chloro-3 (chloro-7 naphtyridine-1,8 yl-2)-2 isoindolinone-1 dans 60 cm3 de diméthylformamide anhydre, de 4,3 g de chlorhydrate de l'acide isopropyl-1 pipéridine-4 carboxylique et de 7,1 g de diaza-1,8 bicyclo [5.4.0] undécène-7. On obtient, après recristallisation dans l'acétonitrile, 4,1 g d'un solide que l'on dissout dans 120 cm3 d'éthanol à reflux ; on ajoute 1,03 g d'acide fumarique en solution dans 20 cm3 d'éthanol. Après cristallisation dans l'acétonitrile, on obtient 4,9 g de fumarate acide d'(isopropyl-1 pipéridyl-4) carboxylate de (chloro-7 naphtyridine-1,8 yl-2)-2 oxo-3 isoindolinyle-1 fondant à 184 ° C.

Le chlorhydrate de l'acide isopropyl-1 pipéridine-4 carboxylique peut être préparé selon la méthode décrite à l'exemple 4 pour la préparation du chlorhydrate de l'acide méthyl-1 pipéridine-3 carboxylique mais à partir de 6,5 g de chlorhydrate d'isopropyl-1 pipéridine-4 carboxylate d'éthyle et de 18,6 cm3 d'une solution aqueuse d'acide chlorhydrique 6N. On obtient ainsi 4,5 g de chlorhydrate de l'acide isopropyl-1 pipéridine-4 carboxylique fondant à 260-265 ° C.

Le chlorhydrate d'isopropyl-1 pipéridine-4 carboxylate d'éthyle peut être préparé de la façon suivante : A une solution de 6,15 g de bromo-2 propane dans 50 cm3 de propanol, on ajoute, en 10 minutes, 15,7 de pipéridine-4 carboxylate d'éthyle à une température voisine de 25 ° C. Le mélange réactionnel est chauffé pendant 48 heures à reflux puis concentré à sec sous pression réduite (2,7 kPa). Le résidu est repris par 150 cm3 d'une solution aqueuse d'acide chlorhydrique 1,5 N et la solution obtenue est lavée par 2 fois 100 cm3 d'éther éthylique. La phase aqueuse est alcalinisée à un pH voisin de 9 par une solution aqueuse de soude 4N, extraite avec 3 fois 100 cm3 de chlorure de méthylène. La phase organique est lavée à l'eau, séchée et concentrée à sec sous pression réduite (2,7 kPa) à 40 ° C. Le résidu obtenu est mis en solution dans 30 cm3 d'éthanol. A la solution obtenue, on ajoute 12 cm3 d'une solution 4,5N d'acide chlorhydrique gazeux dans l'éther éthylique. Le précipité formé est séparé par filtration, lavé et séché. On obtient ainsi 6,9 g de chlorhydrate d'isopropyl-1 pipéridyl-4 carboxylate d'éthyle fondant à 195-200 ° C.

EXEMPLE 17

A une solution de 3,9 g de N-acétyl-β-alanine dans 66 cm3 de diméthylformamide, on ajoute 6,6 g de chloro-3 (chloro-7 naphtyridine-1,8 yl-2)-2 isoindolinone-1. A la suspension beige obtenue, on ajoute, goutte à, 3,8 g de diaza-1,8 bicyclo [5.4.0] undécène-7 et on agite, à une température voisine de 20 ° C, pendant 18 heures. On ajoute à nouveau 1 g de N-acétyl-β-alanine puis 1 g de diaza-1,8 bicyclo [5.4.0] undécène-7 et on agite, à une température voisine de 20 ° C, pendant 24 heures. Le mélange réactionnel est alors versé dans 130 cm3 d'eau. le solide obtenu est séparé par filtration, lavé avec 3 fois 25 cm3 d'eau et purifié par chromatographie sous pression (50 kPa) sur 500 g de silice contenus dans une colonne de 5 cm de diamètre [éluant : dichlorométhane-méthanol (95-5 en volumes)] en recueillant des fractions de 75 cm3. Les

fractions 7 à 9 sont réunies et concentrées à sec sous pression réduite (2,7 kPa). Après recristallisation du solide obtenu dans l'acétonitrile, on obtient 3,9 g d'acétamido-3 propanoate de (chloro-7 naphtyridine-1,8 yl-2)-2 oxo-3 isoindolinyle-1 fondant à 220° C.

La N-acétyl-β-alanine peut être préparée selon la méthode décrite à l'exemple 14 pour la préparation de l'acide propionamido-4 butanoïque mais à partir de 8,9 g de β-alanine, de 9,6 g d'anhydride acétique. On obtient ainsi 12,9 g de N-acétyl-β-alanine à l'état d'huile employée brute dans les synthèses ultérieures.

EXEMPLE 18

On opère comme à l'exemple 24 mais à partir de 5 g d'acide acétamido-5 pentanoïque dans 66 cm3 de diméthylformamide, de 6,6 g de chloro-3 (chloro-7 naphtyridine-1,8 yl-2)-2 isoindolinone-1 et de 4,7 cm3 de diaza-1,8 bicyclo [5.4.0] undécène-7. Après recristallisation dans l'acétonitrile, on obtient 3,3 g d'acétamido-5 pentanoate de (chloro-7 naphtyridine-1,8 yl-2)-2 oxo-3 isoindolinyle-1 fondant à 185° C.

L'acide acétamido-5 pentanoïque peut être préparé selon la méthode décrite à l'exemple 14 pour la préparation de l'acide propionamido-4 butanoïque mais à partir de 11,7 g d'acide amino-5 pentanoïque et de 12,6 g d'anhydride acétique. Le mélange réactionnel est concentré à sec sous pression réduite (0,05 kPa). On obtient ainsi 6 g d'acide acétamido-5 pentanoïque sous forme d'une huile employée brute dans les synthèses ultérieures.

EXEMPLE 19

On opère comme à l'exemple 1 mais à partir de 9,9 g de chloro-3 (chloro-7 naphtyridine-1,8 yl-2)-2 isoindolinone-1 dans 100 cm3 de diméthylformamide anhydre, de 5,2 g d'acide isobutyrylamino-4 butyrique et de 4,6 g de diaza-1,8 bicyclo [5.4.0] undécène-7. Le résidu obtenu est recristallisé dans l'acétonitrile puis purifié par chromatographie sur 130 g de silice (0,063-0,2 mm) contenus dans une colonne de 3 cm de diamètre [éluant : mélange chlorure de méthylène-méthanol (98-2 en volumes)] en recueillant des fractions de 25 cm3. Les fractions 79 à 85 sont réunies et concentrées à sec sous pression réduite (2,7 kPa). Le résidu obtenu est cristallisé par agitation dans l'oxyde d'isopropyle. On obtient ainsi 5,5 g d'isobutyrylamino-4 butyrate de (chloro-7 naphtyridine-1,8 yl-2)-2 oxo-3 isoindolinyle-1 fondant à 208° C.

L'acide isobutyrylamino-4 butyrique peut être préparé selon la méthode décrite à l'exemple 14 pour la préparation de l'acide propionamido-4 butanoïque mais à partir de 10,3 g d'acide amino-4 butyrique et de 15,8 g d'anhydride isobutyrique. On obtient ainsi 15,5 g d'acide isobutyrylamino-4 butyrique à l'état d'huile employée brute dans les synthèses ultérieures.

EXEMPLE 20

A une suspension de 9,9 g de chloro-3 (chloro-7 naphtyridine-1,8 yl-2)-2 isoindolinone-1 et de 6,7 g de chlorhydrate de l'acide diisopropylamino-4 butyrique dans 100 cm3 de diméthylformamide, on ajoute, goutte à goutte, 10,7 g de diaza-1,8 bicyclo [5.4.0] undécène-7 et on agite, à une température voisine de 20° C, pendant 20 heures. On verse alors le mélange réactionnel dans 130 cm3 d'eau. Le solide obtenu est séparé par filtration puis dissous dans 200 cm3 de chlorure de méthylène. Les extraits organiques sont lavés avec 2 fois 35 cm3 d'une solution aqueuse d'acide chlorhydrique 0,1N, 35 cm3 de solution aqueuse d'acide chlorhydrique 0,5N puis avec 2 fois 35 cm3 d'eau. La phase organique est séchée sur sulfate de magnésium, filtrée et concentrée à sec sous pression réduite (2,7 kPa). le résidu obtenu est repris par 200 cm3 de chlorure de méthylène. La phase organique est lavée 2 fois avec 50 cm3 d'une solution aqueuse saturée d'hydrogénocarbonate de sodium. La phase organique est séchée sur sulfate de magnésium, filtrée et concentrée à sec sous pression réduite (2,7 kPa). le résidu est recristallisé dans l'éther isopropylique. On obtient ainsi 7,1 g de diisopropylamino-4 butyrate de (chloro-7 naphtyridine-1,8 yl-2)-2 oxo-3 isoindolinyle-1 fondant à 118° C.

Le chlorhydrate de l'acide diisopropylamino-4 butyrique peut être obtenu en opérant selon la méthode décrite à l'exemple 4 pour la préparation du chlorhydrate de l'acide (méthyl-1 pipéridine-3) carboxylique mais à partir de 9,2 g de diisopropylamino-4 butyrate d'éthyle et de 28,5 cm3 d'une solution aqueuse d'acide chlorhydrique 6N et en chauffant pendant 6 heures au reflux. On obtient ainsi 8 g du chlorhydrate de l'acide diisopropylamino-4 butyrique fondant à 136° C.

Le diisopropylamino-4 butyrate d'éthyle peut être obtenu de la façon suivante : A une solution de 39 g de bromo-4 butyrate d'éthyle dans 80 cm3 d'éthanol, on ajoute, goutte à goutte, 40,4 g de diisopropylamine. La solution obtenue est chauffée à reflux pendant 6 heures. Après filtration de l'insoluble formé, le filtrat est concentré à sec sous pression réduite (2,7 kPa). Le résidu est repris par 15 cm3 d'eau distillée et 100

cm3 d'une solution aqueuse d'acide chlorhydrique 4N. La phase aqueuse est lavée par 3 fois 75 cm3 d'éther éthylique puis alcalinisée par une solution aqueuse de soude 10N. L'huile formée est extraite par 3 fois 75 cm3 d'éther éthylique. La phase organique obtenue est concentrée à sec sous pression réduite (2,7 kPa). On obtient ainsi 9,2 g de diisopropylamino-4 butyrate d'éthyle à l'état d'huile employée brute dans les synthèses ultérieures.

EXEMPLE 21

A une suspension de 12,5 g de (chloro-7 naphtyridine-1,8 yl-2)-2 hydroxy-3 isoindolinone-1 dans 600 cm3 de chlorure de méthylène, on ajoute 12,2 g de triéthylamine, 90 cm3 de pyridine puis 9,7 g de chlorure de méthyl-3 butyryle en maintenant la température aux environs de 25° C. Après 4 heures d'agitation à une température voisine de 25° C, on ajoute à nouveau 9,7 g de chlorure de méthyl-3 butyryle et on agite encore pendant 16 heures à cette température. Le mélange réactionnel est ensuite concentré à sec sous pression réduite (3 kPa) et le résidu est repris par 500 cm3 d'eau. Le produit insoluble formé est séparé par filtration, lavé, séché puis recristallisé dans un mélange d'oxyde d'isopropyle et d'acétate d'éthyle (50-50 en volumes). On obtient ainsi 7,9 g de méthyl-3 butyrate de (chloro-7 naphtyridine-1,8 yl-2)-2 oxo-3 isoindolinyle-1 fondant à 154° C.

EXEMPLE 22

A une solution de 3,2 g de (chloro-7 naphtyridine-1,8 yl-2)-2 hydroxy-3 isoindolinone-1 dans 160 cm3 de chlorure de méthylène, on ajoute successivement 24 cm3 de pyridine puis, en 30 minutes, 5,5 g de chlorure de propionyle en maintenant la température au voisinage de 25° C. Le mélange est agité pendant 3 heures à cette température puis additionné de 100 cm3 d'eau. La phase organique est décantée, lavée à l'eau, séchée et concentrée à sec sous pression réduite (2,7 kPa). Le résidu obtenu est cristallisé dans l'oxyde d'isopropyle puis recristallisé dans un mélange d'oxyde d'isopropyle et d'acétate d'éthyle (25-75 en volumes). On obtient ainsi 1 g de propionate de (chloro-7 naphtyridine-1,8 yl-2)-2 oxo-3 isoindolinyle-1 fondant à 160° C.

EXEMPLE 23

On opère comme à l'exemple 22 mais à partir de 6,4 g de (chloro-7 naphtyridine-1,8 yl-2)-2 hydroxy-3 isoindolinone-1 dans 320 cm3 de chlorure de méthylène, de 48 cm3 de pyridine et de 12,8 g de chlorure de butyryle. Après une recristallisation dans l'oxyde d'isopropyle puis deux recristallisations dans l'acétonitrile, on obtient 3,4 g de butyrate de (chloro-7 naphtyridine-1,8 yl-2)-2 oxo-3 isoindolinyle-1 fondant à 140° C.

EXEMPLE 24

On opère comme à l'exemple 7 mais à partir de 8,55 g de (bromo-7 naphtyridine-1,8 yl-2)-2 hydroxy-3 isoindolinone-1 dans 110 cm3 de chlorure de méthylène, de 11,1 g de triéthylamine, de 7 g de chlorure de méthyl-5 hexanoyle et de 50 mg de diméthylamino-4 pyridine. Après cristallisation dans l'hexane puis recristallisation dans l'éthanol du résidu obtenu, on obtient 7,5 g de méthyl-5 hexanoate de (bromo-7 naphtyridine-1,8 yl-2)-2 oxo-3 isoindolinyle-1 fondant à 136° C.

Le (bromo-7 naphtyridine-1,8 yl-2)-2 hydroxy-3 isoindolinone-1 peut être préparé comme décrit dans le brevet belge 815 019.

EXEMPLE 25

A une suspension de 6,15 g d'hydroxy-3 (méthoxy-7 naphtyridine-1,8 yl-2)-2 isoindolinone-1 dans 50 cm3 de diméthylformamide anhydre, on ajoute, en 15 minutes, 0.96 g d'une suspension huileuse (50 % en poids) d'hydrure de sodium en maintenant la température aux environs de 0° C puis en agitant à nouveau pendant 30 minutes à 0° C. On ajoute ensuite, goutte à goutte, en 30 minutes, 2,9 cm3 de chlorophosphate de diéthyle en maintenant la température aux environs de 0° C. A la solution obtenue, on ajoute à une température voisine de 0° C, une solution d'acétylamino-4 butanoate de sodium préparée à partir de 2,9 g d'acide acétamido-4 butyrique dans 30 cm3 de diméthylformamide anhydre et 0,96 g d'une suspension huileuse (50 % en poids) d'hydrure de sodium. Le mélange est agité pendant 1 heure à 0° C puis 20 heures à une température voisine de 20° C et enfin 4 heures à 80° C. Le mélange réactionnel est versé

dans 400 cm3 d'eau et extrait par 3 fois 200 cm3 de chlorure de méthylène. Les extraits organiques sont réunis et lavés à l'eau, séchés et concentrés à sec sous pression réduite (2,7 kPa). Le résidu obtenu est purifié par chromatographie sur 350 g de silice (0,063-0,2 mm) contenus dans une colonne de 5 cm de diamètre [éluant : chlorure de méthylène-méthanol (99-1 en volumes)] en recueillant des fractions de 100 cm3. Les fractions 23 à 39 sont réunies et concentrées à sec sous pression réduite (2,7 kPa) à 40 °C ; le résidu est recristallisé dans l'acétonitrile. On obtient ainsi 2 g d'acétylamino-4 butyrate de (méthoxy-7 naphtyridine-1,8 yl-2)-2 oxo-3 isoindolinyle-1 fondant à 190 °C.

EXEMPLE 26

On opère comme à l'exemple 7 mais à partir de 3,7 g de (méthyl-7 naphtyridine-1,8 yl-2)-2 hydroxy-3 isoindolinone-1, de 7,8 g de chlorure de méthyl-5 hexanoyle, de 12 cm3 de triéthylamine et de 50 mg de diméthylamino-4 pyridine. Après recristallisation dans le méthylcyclohexane du solide obtenu après traitement, on obtient 3,8 g de méthyl-5 hexanoate de (méthyl-7 naphtyridine-1,8 yl-2)-2 oxo-3 isoindolinyle-1 fondant à 144 °C.

L'hydroxy-3 (méthyl-7 naphtyridine-1,8 yl-2)-2 isoindolinone-1 peut être préparée par la méthode décrite dans le brevet allemand 2 423 650.

Le chlorure de méthyl-5 hexanoyle peut être préparé par la méthode décrite par GOERNER G.L. et Coll., J. Org. Chem., 24, 1561 (1959).

EXEMPLE 27

On opère comme à l'exemple 1 mais à partir de 9,9 g de chloro-3 (chloro-7 naphtyridine-1,8 yl-2)-2 isoindolinone-1 dans 100 cm3 de diméthylformamide anhydre, de 4,8 g d'acide (N-méthyl-acétamido)-4butanoïque et de 4,6 g de diaza-1,8 bicyclo [5.4.0] undécène-7. Le résidu huileux obtenu est purifié par chromatographie sur 150 g de silice (0,063 - 0,2 mm) contenus dans une colonne de 3 cm de diamètre (éluant : chlorure de méthylène - méthanol (99-1 en volumes)] et en recueillant des fractions de 30 cm3. Les fractions 76 à 175 sont concentrées à sec sous pression réduite (2,7 kPa) à 60 °C et après cristallisation dans l'éther éthylique, on obtient 3,1 g de (N-méthyl-acétamido)-4 butyrate de (chloro-7 naphtyridine-1,8 yl-2)-2 oxo-3 isoindolinyle-1 fondant à 170 °C.

L'acide (N-méthyl-acétamido)-4 butanoïque peut être préparé de la façon suivante : A une solution de 15,4 g de chlorhydrate de l'acide (N-méthyl-amino)-4 butanoïque dans 200 cm3 d'une solution aqueuse de soude 2,5N, maintenue à une température voisine de 5 °C, on ajoute, en 1 heure, 11,8 g de chlorure d'acètyle. Le mélange est agité à nouveau pendant 30 minutes aux environs de 5 °C puis acidifié à un pH voisin de 1 à l'aide d'une solution aqueuse d'acide chlorhydrique 12N. Le mélange est concentré à sec à 80 °C sous pression réduite (2,7 kPa) et le résidu obtenu est repris par 150 cm3 d'éthanol. Un solide est séparé par filtration et le filtrat est concentré à sec à 60 °C sous pression réduite, puis le résidu est repris par 150 cm3 de chlorure de méthylène. La phase organique est séchée sur sulfate de magnésium et concentrée à sec à 60 °C sous pression réduite (2,7 kPa). On obtient ainsi 19 g d'acide (N-méthyl-acétamido)-4 butanoïque à l'état d'huile employée brute dans les synthèses ultérieures.

EXEMPLE 28

On opère comme à l'exemple 7 mais à partir de 5 g de (fluoro-7 naphtyridine-1,8 yl-2)-2 hydroxy-3 isoindolinone-1 dans 60 cm3 de chlorure de méthylène, de 6,5 g de triéthylamine, de 3,66 g de chlorure de méthyl-4 pentanoyle et de 10 mg de diméthylamino-4 pyridine. On obtient, après recristallisation dans l'éthanol, 1,2 g de méthyl-4 pentanoate de (fluoro-7 naphtyridine-1,8 yl-2)-2 oxo-3 isoindolinyle-1 fondant à 154 °C.

La (fluoro-7 naphtyridine-1,8 yl-2)-2 hydroxy-3 isoindolinone-1 peut être préparée de la manière suivante : A une suspension de 16,6 g de (fluoro-7 naphtyridine-1,8 yl-2)-2 isoindolinedione-1,3 dans un mélange de 90 cm3 de méthanol anhydre et de 90 cm3 de dioxanne, on ajoute, à une température voisine de 20 °C, par petites portions, 2,3 g de tétrahydruroborate de potassium, et agite la suspension obtenue pendant 3 heures à une température voisine de 20 °C. Le mélange réactionnel est ensuite versé dans un mélange de 120 g de glace et 240 cm3 d'eau. Le produit insoluble est séparé par filtration, lavé par 3 fois 50 cm3 d'eau, séché à l'air et recristallisé dans l'acétonitrile. On obtient ainsi 10,3 g de (fluoro-7 naphtyridine-1,8 yl-2)-2 hydroxy-3 isoindolinone-1 fondant à 246 °C.

La (fluoro-7 naphtyridine-1,8 yl-2)-2 isoindolinedione-1,3 peut être préparée de la manière suivante : A une suspension de 20,6 g de (chloro-7 naphtyridine-1,8 yl-2)-2 isoindolinedione-1,3 dans 270 cm3 de

nitrobenzène anhydre, maintenue sous atmosphère d'argon, on ajoute 15 g de fluorure de potassium et chauffe le mélange réactionnel à reflux sous agitation, pendant 22 heures. Après refroidissement à une température voisine de 80° C, le mélange réacttionnel est concentré à sec sous pression réduite (0.13 kPa) à 80° C. Le résidu obtenu est repris par 170 cm3 d'acétate d'éthyle. Le produit insoluble est séparé par filtration, lavé successivement par 30 cm3 d'acétate d'éthyle, 6 fois 30 cm3 d'eau et séché à l'air. On obtient ainsi 16,9 g de (fluoro-7 naphtyridine-1,8 yl-2)-2 isoindolinedione-1,3 fondant à 264° C.

La (chloro-7 naphtyridine-1,8 yl-2)-2 isoindolinedione-1,3 peut être préparée par la méthode décrite dans le brevet belge 835 325.

La présente invention concerne également les médicaments qui contiennent les produits de formule (I) à l'état pur ou sous forme de compositions dans lesquelles ils sont associés à un adjuvant, un diluant et/ou un enrobage compatible et pharmaceutiquement acceptable. Ces médicaments peuvent être employés par voie orale, rectale, parentérale ou percutanée.

Comme compositions solides pour administration orale peuvent être utilisés des comprimés, des pilules, des poudres (généralement dans des capsules de gélatine) ou des granulés. Dans ces compositions, le produit actif selon l'invention est mélangé à un ou plusieurs diluants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple, des produits mouillants, édulcorants ou aromatisants.

Les compositions selon l'invention pour administration parentérale peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive ou des esters organiques injectables, par exemple l'oléate d'éthyle. Ces compositions peuvent également contenir des adjuvants, en particulier des agents mouillants, émulsifiants et dispersants. La stérilisation peut se faire de plusieurs façons, par exemple à l'aide d'un filtre bactériologique, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont des suppositoires qui peuvent contenir, outre le produit actif, des excipients tels que le beurre de cacao ou la suppo-cire.

Les compositions pour administration percutanée sont les crèmes, pommades, lotions et liniments, dans lesquels le produit actif est associé à des excipients liquides ou pâteux, de préférence en association avec un véhicule favorisant la migration percutanée.

Les médicaments et compositions selon l'invention sont particulièrement utiles en thérapeutique humaine pour leur action anxiolytique, hypnotique, anticonvulsivante, antiépileptique et myorelaxante.

En thérapeutique humaine, les doses dépendent de l'effet recherché et de la durée du traitement; elles sont généralement comprises entre 10 et 500 mg par jour par voie orale pour un adulte.

D'une façon générale, le médecin déterminera la posologie qu'il estime la plus appropriée en fonction de l'âge, du poids et de tous les autres facteurs propres au sujet à traiter.

Les exemples suivants illustrent une composition selon l'invention.

Exemple A

On prépare selon la technique habituelle des comprimés dosés à 10 mg de produit actif ayant la composition suivante :

- diméthylamino-3 propionate de (chloro-7 naphtyridine-1,8 yl-2)-2 oxo-1 isoindolinyle-1 .................... 0,010 g
- amidon .................... 0,200 g
- silice précipitée .................... 0,036 g
- stéarate de magnésium .................... 0,004 g

En opérant de la même manière, on peut préparer des comprimés dont le principe actif est constitué des produits suivants :

- Acétamido-4 butyrate de (chloro-7 naphtyridine-1,8 yl-2)-2 oxo-3 isoindolinyle-1-(RS)
- (Propionyl-1 pipéridyl-4) carboxylate de (chloro-7 naphtyridine-1,8 yl-2)-2 oxo-3 isoindolinyle-1-(RS)
- Méthyl-5 hexanoate de (méthoxy-7 naphtyridine-1,8 yl-2)-2 oxo-3 isoindolinyle-1-(RS)
- Diisopropylamino-3 propionate de (chloro-7 naphtyridine-1,8 yl-2)-2 oxo-3 isoindolinyle-1-(RS).

**Revendications**

Revendications pour les Etats Contractants suivants : AT, BE, CH, DE, FR, GB, IT, LI, LU, NL, SE

**1.** Nouveaux dérivés du pyrrole, caractérisés en ce qu'ils répondent à la formule générale :

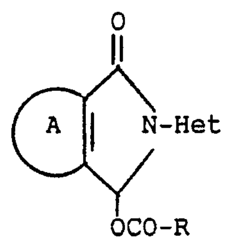

(I)

dans laquelle A forme avec le cycle pyrrole un noyau isoindoline et Het représente un radical naphtyridinyle substitué par un atome d'halogène ou un radical alcoyle (1 à 4 C), alcoyloxy (1 à 4 C) et R représente un radical alcoyle non substitué ou substitué par un radical alcoyloxy, dialcoylamino, alcoylcarbonylamino (dont la partie amino peut être éventuellement substituée par un radical alcoyle), pipéridyle ou pipéridino ou bien R représente un radical pipéridyle-4, étant entendu que les radicaux alcoyle sont en chaîne droite ou ramifiée et contiennent, sauf mention spéciale, 1 à 10 atomes de carbone et que les radicaux pipéridino, pipéridyle peuvent être non substitués ou substitués en position quelconque par un radical alcoyle, ainsi que, lorsqu' ils existent, leurs sels pharmaceutiquement acceptables et les isomères optiques des produits de formule (I).

**2.** Procédé de préparation d'un produit selon la revendication 1, dans laquelle Het est défini comme à la revendication 1 à l'exception de représenter un radical naphtyridine-1,8 yle-2 substitué en position 7 par un radical alcoyloxy, caractérisé en ce que l'on fait agir un acide de formule générale :

R-COOH (II)

ou un sel de métal alcalin de cet acide dans laquelle R est défini comme à la revendication 1, sur un produit de formule générale :

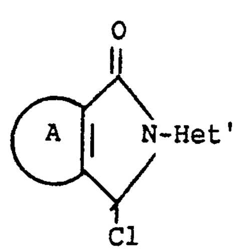

(III)

dans laquelle Het' est défini comme Het à la revendication 1 à l'exception de représenter un radical naphtyridine-1,8 yle-2 substitué en position 7 par un radical alcoyloxy et A est défini comme à la revendication 1, puis isole le produit obtenu et le transforme éventuellement, le cas échéant, en un sel pharmaceutiquement acceptable.

**3.** Procédé de préparation d'un produit selon la revendication 1, caractérisé en ce que l'on fait agir un produit de formule générale :

RCO-X (V)

dans laquelle R est défini comme à la revendication 1 et X représente un atome d'halogène ou un reste d'ester réactif sur un produit de formule générale :

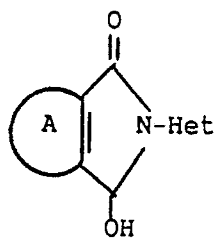

(IV)

dans laquelle A et Het sont définis comme à la revendication 1, puis isole le produit obtenu et le transforme éventuellement, le cas échéant, en un sel pharmaceutiquement acceptable.

**4.** Procédé de préparation d'un produit selon la revendication 1, caractérisé en ce que l'on fait réagir un sel alcalin d'un acide de formule générale :

R-COOH (II)

dans laquelle R est défini comme à la revendication 1, sur un produit de formule générale :

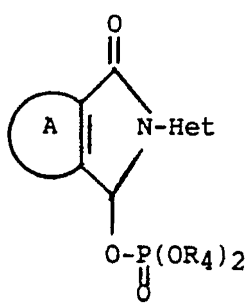

(VI)

dans laquelle $R_4$ représente un radical alcoyle contenant 1 à 4 atomes de carbone en chaîne droite ou ramifiée ou un radical phényle et A et Het sont définis comme à la revendication 1, puis isole le produit obtenu et le transforme si on le désire et le cas échéant, en un sel pharmaceutiquement acceptable.

**5.** Composition pharmaceutique, caractérisée en ce qu'elle contient au moins un produit selon la revendication 1, en association avec un ou plusieurs diluants compatibles et pharmaceutiquement acceptables.

Revendication pour les Etats Contractants suivants : ES, GR

**1.** Procédé de préparation de nouveaux dérivés du pyrrole de formule générale :

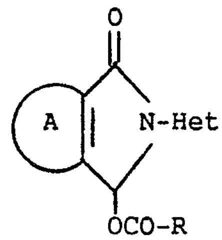

(I)

dans laquelle A forme avec le cycle pyrrole un noyau isoindoline et Het représente un radical naphtyridinyle substitué par un atome d'halogène ou un radical alcoyle (1 à 4 C), alcoyloxy (1 à 4 C) et R représente un radical alcoyle non substitué ou substitué par un radical alcoyloxy, dialcoylamino, alcoylcarbonylamino (dont la partie amino peut être éventuellement substituée par un radical alcoyle), pipéridyle ou pipéridino, ou bien R représente un radical pipéridyle-4, étant entendu que les radicaux alcoyle sont en chaîne droite ou ramifiée et contiennent, sauf mention spéciale, 1 à 10 atomes de carbone et que les radicaux pipéridino, pipéridyle peuvent être non substitués ou substitués en position quelconque par un radical alcoyle, ainsi que, lorsqu'ils existent, leurs sels pharmaceutiquement acceptables et les isomères optiques des produits de formule (I), caractérisé en ce que :

A. Pour la préparation d'un produit de formule générale (I), dans laquelle Het est défini comme précédemment à l'exception de représenter un radical naphtyridine-1,8 yle-2 substitué en position 7 par un radical alcoyloxy, on fait agir un acide de formule générale :

R-COOH (II)

ou un sel de métal alcalin de cet acide dans laquelle R est défini comme précédemment, sur un produit de formule générale :

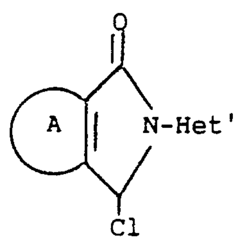

(III)

dans laquelle Het' est défini comme Het précédemment à l'exception de représenter un radical naphtyridine-1,8 yle-2 substitué en position 7 par un radical alcoyloxy et A est défini comme précédemment, puis isole le produit obtenu et le transforme éventuellement, le cas échéant, en un sel pharmaceutiquement acceptable, ou en ce que :

B. Pour la préparation d'un produit de formule générale (I) défini comme précédemment, on fait agir un produit de formule générale :

RCO-X (V)

dans laquelle R est défini comme précédemment et X représente un atome d'halogène ou un reste d'ester réactif sur un produit de formule générale :

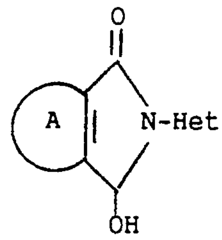

(IV)

dans laquelle A et Het sont définis comme précédemment, puis isole le produit obtenu et le transforme éventuellement, le cas échéant, en un sel pharmaceutiquement acceptable, ou en ce que :

C. Pour la préparation d'une produit comme précédemment, on fait réagir un sel alcalin d'un acide de formule générale :

R-COOH (II)

dans laquelle R est défini comme précédemment, sur un produit de formule générale :

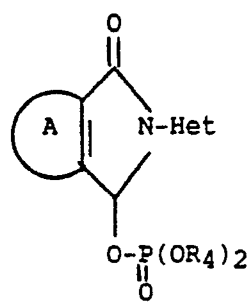

(VI)

dans laquelle $R_4$ représente un radical alcoyle contenant 1 à 4 atomes de carbone en chaîne droite ou ramifiée ou un radical phényle et A et Het sont définis comme précédemment, puis isole le produit obtenu et le transforme si on le désire et le cas échéant, en un sel pharmaceutiquement acceptable.

**Claims**

Claims for the following Contracting States : AT, BE, CH, DE, FR, GB, IT, LI, LU, NL, SE

**1.** A new pyrrole derivative which is of the general formula:

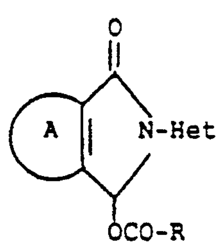

(I)

in which A forms with the pyrrole ring an isoindoline ring-system, Het denotes a naphthyridinyl radical substituted with a halogen atom or a (1 to 4 C) alkyl or (1 to 4 C) alkyloxy radical and R denotes an alkyl radical which is unsubstituted or substituted with an alkyloxy or dialkylamino radical, with an alkylcarbonylamino radical (in which the amino portion can optionally be substituted with an alkyl radical), or with a piperidyl or piperidino radical, or alternatively R denotes a 4-piperidyl radical, on the understanding that the alkyl radicals are straight- or branched-chain radicals and contain, except where

specifically stated, 1 to 10 carbon atoms, and that the piperidino and piperidyl radicals can be unsubstituted or substituted at any position with an alkyl radical, as well as, where they exist, its pharmaceutically acceptable salts and the optical isomers of the products of formula (I).

2. A process for preparing a product according to claim 1, in which Het is defined as in claim 1 with the exception of denoting a 1,8-naphthyridin-2-yl radical substituted at the 7-position with an alkyloxy radical, wherein an acid of general formula:

R-COOH (II)

or an alkali metal salt of this acid, in which R is defined as in claim 1, is reacted with a product of general formula:

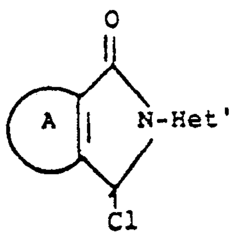
(III)

in which Het' is defined as Het in claim 1, with the exception of denoting a 1,8-naphthyridin-2-yl radical substituted at the 7-position with an alkyloxy radical, and A is defined as in claim 1, and the product obtained is then isolated and optionally converted, where appropriate, to a pharmaceutically acceptable salt.

3. A process for preparing a product according to claim 1, wherein a product of general formula:

RCO-X (V)

in which R is defined as in claim 1 and X denotes a halogen atom or a reactive ester residue, is reacted with a product of general formula:

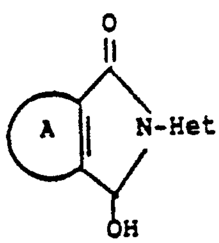
(IV)

in which A and Het are defined as in claim 1, and the product obtained is then isolated and optionally converted, where appropriate, to a pharmaceutically acceptable salt.

4. A process for preparing a product according to claim 1, wherein an alkali metal salt of an acid of general formula:

R-COOH (II)

in which R is defined as in claim 1, is reacted with a product of general formula:

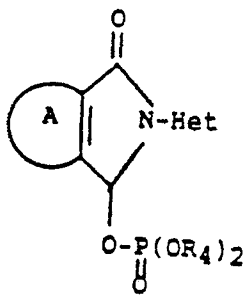

(VI)

in which $R_4$ denotes a straight- or branched-chain alkyl radical containing 1 to 4 carbon atoms or a phenyl radical and A and Het are defined as in claim 1, and the product obtained is then isolated and converted, if so desired and where appropriate, to a pharmaceutically acceptable salt.

**5.** A pharmaceutical composition which contains at least one product according to claim 1, in combination with one or more diluents which are compatible and pharmaceutically acceptable.

Claim for the following Contracting States : ES, GR

**1.** A process for preparing a new pyrrole derivative of general formula:

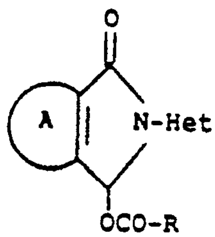

(I)

in which A forms with the pyrrole ring an isoindoline ring-system, Het denotes a naphthyridinyl radical substituted with a halogen atom or a (1 to 4 C) alkyl or (1 to 4 C) alkyloxy radical and R denotes an alkyl radical which is unsubstituted or substituted with an alkyloxy or dialkylamino radical, with an alkylcarbonylamino radical (in which the amino portion can optionally be substituted with an alkyl radical), or with a piperidyl or piperidino radical, or alternatively R denotes a 4-piperidyl radical, on the understanding that the alkyl radicals are straight- or branched-chain radicals and contain, except where specifically stated, 1 to 10 carbon atoms, and that the piperidino or piperidyl radicals can be unsubstituted or substituted at any position with an alkyl radical, as well as, where they exist, its pharmaceutically acceptable salts and the optical isomers of the products of formula (I), wherein:

A. For the preparation of a product of general formula I, in which Het is defined as above with the exception of denoting a 1,8-naphthyridin-2-yl radical substituted at the 7-position with an alkyloxy radical, an acid of general formula:

R-COOH (II)

or an alkali metal salt of this acid, in which R is defined as above, is reacted with a product of general formula:

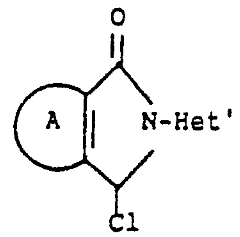

(III)

in which Het' is defined as Het above, with the exception of denoting a 1,8-naphthyridin-2-yl radical substituted at the 7-position with an alkyloxy or alkylthio radical, and A is defined as above and the product obtained is then isolated and optionally converted, where appropriate, to a pharmaceutically acceptable salt, or wherein:

B. For the preparation of a product of general formula (I) defined as above, a product of general formula:

RCO-X (V)

in which R is defined as above and X denotes a halogen atom or a reactive ester residue, is reacted with a product of general formula:

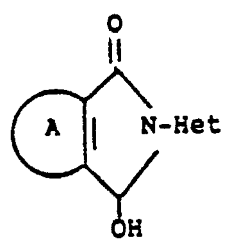

(IV)

in which A and Het are defined as above, and the product obtained is then isolated and optionally converted, where appropriate, to a pharmaceutically acceptable salt, or wherein:

C. For the preparation of a product as above, an alkali metal salt of an acid of general formula:

R-COOH (II)

in which R is defined as above, is reacted with a product of general formula:

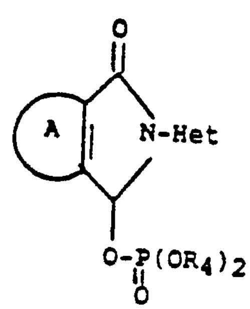

(VI)

in which $R_4$ denotes a straight- or branched-chain alkyl radical containing 1 to 4 carbon atoms or a phenyl radical and A and Het are defined as above, and the product obtained is then isolated and converted, if so desired and where appropriate, to a pharmaceutically acceptable salt.

**Patentansprüche**

Patentansprüche für folgende Vertragsstaaten : AT BE CH DE FR GB IT LI LU NL SE

1. Neue Pyrrolderivate, dadurch gekennzeichnet, daß sie der allgemeinen Formel:

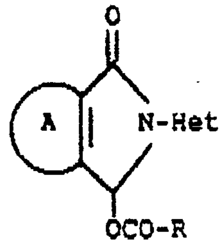

(I)

entsprechen, in welcher A zusammen mit dem Pyrrolring einen Isoindolinkern bildet und Het einen durch ein Halogenatom oder einen (1 bis 4 C) Alkyl-, (1 bis 4 C) Alkyloxyrest substituierten Naphthyridinylrest darstellt und R einen gegebenenfalls durch einen Alkyloxy-, Dialkylamino-, Alkylcarbonylamino-(dessen Aminoteil gegebenenfalls durch einen Alkylrest substituiert sein kann), Piperidyl- oder Piperidinorest substituierten Alkylrest darstellt, oder R einen 4-Piperidylrest darstellt, wobei die Alkylreste selbstverständlich geradkettig oder verzweigt sind und ohne spezielle Angabe 1 bis 10 Kohlenstoffatome enthalten und die Piperidino- Piperidylreste gegebenenfalls in irgendeiner Stellung durch einen Alkylrest substituiert sind, sowie, falls es sie gibt, ihre pharmazeutisch zulässigen Salze und die optischen Isomeren der Verbindungen der Formel (I).

2. Verfahren zur Herstellung einer Verbindung nach Anspruch 1, in deren Formel Het die im Anspruch 1 angegebene Bedeutung mit Ausnahme eines in 7-Stellung durch einen Alkyloxyrest substituiertcn 1,8-Naphthyridin-2-ylrestes hat, dadurch gekennzeichnet, daß man eine Säure der allgemeinen Formel:

R-COOH (II)

oder ein Alkalimetallsalz dieser Säure, worin R die im Anspruch 1 angegebene Bedeutung hat, mit einer Verbindung der allgemeinen Formel:

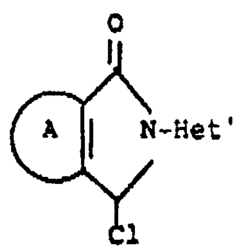

(III)

in welcher Het' die im Anspruch 1 für Het angegebene Bedeutung mit Ausnahme eines in 7-Stellung durch einen Alkyloxyrezt substituierten 1,8-Naphthyridin-2-ylrestes hat und A die im Anspruch 1 angegebene Bedeutung hat, umsetzt, dann die erhaltene Verbindung isoliert und sie gewünschtenfalls zutreffendenfalls in ein pharmazeutisch zulässiges Salz umwandelt.

3. Verfahren zur Herstellung einer Verbindung nach Anspruch 1, dadurch gekennzeichnet, daß man eine Verbindung der allgemeinen Formel:

RCO-X (V)

in welcher R die im Anspruch 1 angegebene Bedeutung hat und X ein Halogenatom oder einen reaktionsfähigen Esterrest darstellt, mit einer Verbindung der allgemeinen Formel:

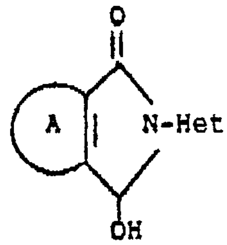

(IV)

in welcher A und Het die im Anspruch 1 angegebene Bedeutung haben, umsetzt, dann die erhaltene Verbindung isoliert und sie gegebenenfalls und zutreffendenfalls in ein pharmazeutisch zulässiges Salz umwandelt.

**4.** Verfahren zur Herstellung einer Verbindung nach Anspruch 1, dadurch gekennzeichnet, daß man ein Alkalisalz einer Säure der allgemeinen Formel:

R-COOH (II)

worin R die im Anspruch 1 angegebene Bedeutung hat, mit einer Verbindung der allgemeinen Formel:

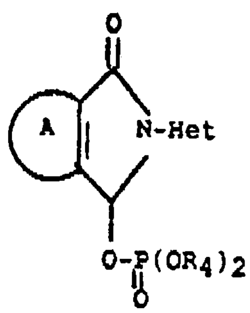

(VI)

in welcher $R_4$ einen Alkylrest mit 1 bis 4 Kohlenstoffatomen in gerader oder verzweigter Kette oder einen Phenylreat darstellt und A und Het die im Anspruch 1 angegebene Bedeutung haben, umsetzt, dann das erhaltene Produkt isoliert und es gewünschtenfalls zutreffendenfalls in ein pharmazeutisch zulässiges Salz umwandelt.

**5.** Pharmazeutische Massen, dadurch gekennzeichnet, daß sie zumindest eine Verbindung nach Anspruch 1 zusammen mit einem oder mehreren verträglichen und pharmazeutisch zulässigen Verdünnungsmitteln oder Zusatzstoffen enthalten.

Patentanspruch für folgende Vertragsstaaten : ES GR

**1.** Verfahren zur Herstellung neuer Pyrrolderivate der allgemeinen Formel:

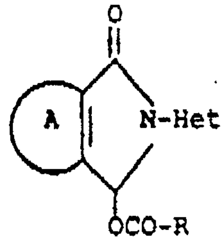

(I)

in welcher A zusammen mit dem Pyrrolring einen Isoindolinkern bildet und Het einen durch ein

Halogenatom oder einen (1 bis 4 C) Alkyl-, (1 bis 4 C) Alkyloxyrest substituierten Naphthyridinylrest darstellt und R einen gegebenenfalls durch einen Alkyloxy-, Dialkylamino-, Alkylcarbonylamino- (dessen Aminoteil gegebenenfalls durch einen Alkylrest substituiert sein kann), Piperidyl- oder Piperidinorest substituierten Alkylrest darstellt, oder R einen 4-Piperidylrest darstellt, wobei die Alkylreste selbstverständlich geradkettig oder verzweigt sind und ohne spezielle Angabe 1 bis 10 Kohlenstoffatome enthalten und die Piperidino- Piperidylreste gegebenenfalls in irgendeiner Stellung durch einen Alkylrest substituiert sind, sowie, falls sie gibt, ihrer pharmazeutisch zulässigen Salze und der optischen Isomeren der Verbindungen der Formel (I),
dadurch gekennzeichnet, daß man

A - zur Herstellung einer Verbindung der allgemeinen Formel (I), in welcher Het die oben angegebene Bedeutung mit Ausnahme eines in 7-Stellung durch einen Alkyloxyrest substituierten 1,8-Naphthyridin-2-ylrestes hat, eine Säure der allgemeinen Formel:

R-COOH (II)

oder ein Alkalimetallsalz dieser Säure, worin R die oben angegebene Bedeutung hat, mit einer Verbindung der allgemeinen Formel:

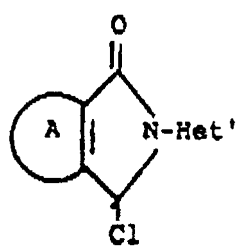

(III)

in welcher Het' die oben für Het angegebene Bedeutung mit Ausnahme eines in 7-Stellung durch einen Alkyloxyrest substituierten 1,8-Naphthyridin-2-ylrestes hat und A die oben angegebene Bedeutung hat, umsetzt, dann die erhaltene Verbindung isoliert und sie gewünschtenfalls zutreffendenfalls in ein pharmazeutisch zulässiges Salz umwandelt oder daß man

B - zur Herstellung einer Verbindung der oben definierten allgemeinen Formel (I) eine Verbindung der allgemeinen Formel:

RCO-X (V)

in welcher R die oben angegebene Bedeutung hat und X ein Halogenatom oder einen reaktionsfähigen Esterrest darstellt, mit einer Verbindung der allgemeinen Formel:

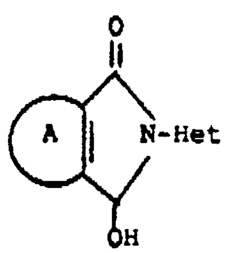

(IV)

in welcher A und Het die oben angegebene Bedeutung haben, umsetzt, dann die erhaltene Verbindung isoliert und sie gegebenenfalls und zutreffendenfalls in ein pharmazeutisch zulässiges Salz umwandelt, oder daß man

C - zur Herstellung einer oben definierten Verbindung ein Alkalisalz einer Säure der allgemeinen Formel:

R-COOH (II)

worin R die oben angegebene Bedeutung hat, mit einer Verbindung der allgemeinen Formel:

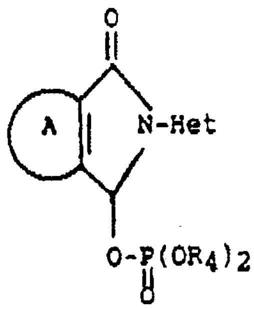

(VI)

in welcher $R_4$ einen Alkylrest mit 1 bis 4 Kohlenstoffatomen in gerader oder verzweigter Kette oder einen Phenylrest darstellt und A und Het die oben angegebene Bedeutung haben, umsetzt, dann das erhaltene Produkt isoliert und es gewünschtenfalls zutreffendenfalls in ein pharmazeutisch zulässiges Salz umwandelt.